# EUROPEAN PATENT SPECIFICATION

(11) **EP 3 785 616 B1**
(45) Date of publication and mention of the grant of the patent: **07.12.2022**
(21) Application number: 19194838.9
(22) Date of filing: 02.09.2019
(51) Int. Cl.: A61B 5/02, A61B 5/0215, A61B 5/107, A61B 5/00

(54) **IMPLANTATION CATHETER**
IMPLANTATIONSKATHETER
CATHÉTER D'IMPLANTATION

(43) Date of publication of application: 03.03.2021
(73) Proprietor: BIOTRONIK SE & Co. KG, 12359 Berlin (DE)
(72) Inventor: Arndt, Andreas, 12555 Berlin (DE); Meine, Sonja, 12681 Berlin (DE); Nollert, Georg, 82064 Strasslach (DE)
(74) Representative: Biotronik Corporate Services SE

(56) References cited:
- WO-A1-2019/055434
- US-A- 5 370 618
- US-A- 5 725 571
- US-A1- 2005 160 827
- US-A1- 2010 087 782
- US-A1- 2017 196 478

## Description

The present disclosure relates to an implantation catheter and to an implantation catheter arrangement.

Implantation catheters are well-known in the prior art. For example, US 2017/0196478 A1, US 2005/160827 A1, US 2010/087782 A1, US 5 370 618 A and US 5 725 571 A disclose catheters with different configurations.

Implantation catheters can be used for delivering implants to an implantation site. Thereby, different implantation catheters are available for different implants to be delivered. Furthermore, the implantation catheters are often specifically designed in dependence on the implantation site to be reached by the implantation catheter.

For implanting vascular implants it is regularly necessary to enter the body of the patient by a peripheral venous access. For current pulmonary artery implants, the right ventricle is passed and an appropriate branch of the pulmonary artery is probed. The aim of this probing is to find an appropriate target diameter of the pulmonary artery for the implant to be implanted.

During the implantation procedure of pulmonary implants in addition to the pulmonary artery pressure, the so called pulmonary artery wedge pressure is commonly measured. This is typically done with a Swan-Ganz catheter. This is a balloon catheter which is entered into the pulmonary artery. Then, the balloon is inflated and wedges in a small pulmonary blood vessel, thereby obstructing this pulmonary blood vessel. The pressure measured distal of the Swan-Ganz catheter in this wedged position is called pulmonary wedge pressure. It is indicative for the pressure present in the left atrium.

The following steps are performed according to established prior art techniques if a sensor is implanted into the pulmonary artery of a patient:
First, a balloon catheter is introduced into the pulmonary artery to measure the pulmonary wedge pressure and/or other hemodynamic parameters such as a pulmonary artery pressure. These measurements are used for baseline comparisons and calibration.

Then, a guide wire is advanced through the patient's vascular system to the intended site of implantation (i.e., the pulmonary artery).

Then, the balloon catheter is removed.

Afterwards, an implantation catheter is advanced with the help of the guide wire. The desired implantation site is thereby determined under X-ray control, typically with prior contrast medium application. Determination of the implant site for current non-powered devices is crucial for the success, because they need to be accessed from the outside, which is only possible in appropriate locations. Pulmonary angiograms are necessary to determine the appropriate vessel diameter and interventional operator skills are needed to steer the guidewire to the appropriate position.

Then, the sensor to be implanted is released from the implantation catheter.

Afterwards, the implantation catheter is removed.

Then, the balloon catheter is again advanced towards the pulmonary artery in order to perform a reference measurement of the pulmonary wedge pressure and/or other hemodynamic parameters.

Subsequently, both the balloon catheter and the guide wire are removed.

Thus, this classic implantation method requires different tools and a repetitive change of these tools to be inserted into the pulmonary artery of the patient. It currently needs an expensive environment with an angiography unit, highly skilled operators, iodine contrast injections and a potentially harmful technique using guidewires to perform the procedure.

If a slim and sharp implantation catheter is navigated through the patient's body - even if this is done with the help of a guide wire -, the risk of vascular injury with subsequent bleeding is present. Furthermore, the risk of injuring structures in the right heart, in particular of the tricuspid or the pulmonary valve, is present.

Furthermore, typically a rather long X-ray exposure time is necessary to find the target position of the implant to be implanted into the pulmonary artery.

It is an object to provide an implantation catheter which facilitates implantation of a vascular implant such as a sensor, in particular into the pulmonary artery.

To that end, the present invention provides an implantation catheter for implanting a vascular implant into a vessel (e.g. the pulmonary artery) of a human or an animal with the features of independent claim 1. Amongst others, the implantation catheter comprises a catheter shaft having a proximal end and a distal end. Additionally, the implantation catheter comprises a vascular implant carried by the catheter shaft. The implantation catheter comprises a pressure sensor for sensing a vascular pressure at its distal end. In an alternative embodiment, not according to the present invention, the implantation catheter may comprise an inner lumen filled with a liquid for pressure measurement. Other sensors may be part of other embodiments, such as for measuring oxygen saturation, temperature, pH etc.

The implantation catheter furthermore comprises an inflatable balloon arranged circumferentially (radially) around an outside of the catheter shaft in a distal end section of the catheter shaft.

Such an implantation catheter does no longer require a guide wire. Rather, it can be guided to the desired implantation site by the blood flow. In doing so, the balloon is inflated up to a desired size. The size of the balloon corresponds to the size of the site of the vessel, in particular of the pulmonary artery, at which the vascular implant is to be implanted. If the vascular implant is to be implanted in the pulmonary artery, the implantation catheter floats due to the inflated balloon automatically after an introduction into the right heart further into the pulmonary artery and then gets stuck in the pulmonary artery at the implantation site. It is no longer necessary to control the advancement of the implantation catheter by X-rays. Rather, it is fully sufficient to control the correct positioning of the implantation catheter once after the balloon has reached its final destination, in particular within the pulmonary artery. No or only a small quantity of iodine contrast is needed to determine the vessel diameter.

The implantation catheter enables a measurement of the pulmonary wedge pressure and further hemodynamic parameters (such as the pulmonary artery pressure) as well as an implantation of a vascular implant with the same device. Thus, it is no longer necessary to change the instruments during implantation of a vascular implant. Thus, it is only necessary to introduce a single implantation catheter into the body of a patient. This significantly reduces the risk of injuries of the patient's vessels. Furthermore, in an embodiment, no guide wire is necessary for the implantation catheter. This additionally reduces the risk of injuries to the patient's vessels. By combining the properties of a pressure-monitoring balloon catheter and the properties of an implantation catheter into a single device, the operational safety and the patients' comfort is thus significantly increased.

In an embodiment, the distal end section extends from the distal end towards the proximal end over up to 10 %, in particular over up to 9 %, in particular over up to 8 %, in particular over up to 7 %, in particular over up to 6 %, in particular over up to 5 %, in particular over up to 4 %, in particular of up to 3 %, in particular over 2 %, in particular over up to 1 % of the total length of the catheter shaft. Then, it is particularly easy to navigate the implantation catheter with an inflated (expanded) balloon through the blood vessels of a patient.

It is not necessary that the balloon extends over the full distal end region. Rather, it is typically arranged in a certain section of the distal end region. The distal end of the balloon flushes with the distal end of the catheter shaft. In this way, the balloon protects the vessels of the patient in a very appropriate manner against potential injuries that might occur due to a contact of the distal end of the catheter shaft and a vessel of the patient.

In an embodiment, the pressure sensor of the implantation catheter is an electronic pressure sensor. Electronic pressure sensors provide very reliable and exact results so that the pressure measured with such a pressure sensor can also considered being highly reliable. An electric line necessary to read out the electronic pressure sensor can be guided through an available lumen of the catheter shaft. Standard catheter shafts typically have a plurality of lumina, wherein often one or more of these lumina are not used by other parts of the implantation catheter so that they are available for such an electric line.

In an embodiment, the implantation catheter comprises a handle. Thereby, this handle may comprise evaluation electronics for reading the pressure sensor out. Thus, the pressure sensor provides the evaluation electronics with the data to be read-out through an electric line, wherein all further calculations for obtaining the measured pressure value can be done by the evaluation electronics housed in the handle of the implantation catheter.

In an embodiment, the evaluation electronics comprises a wireless communication module. This wireless communication module can be used for transferring pressure data sent by the pressure sensor to an external evaluation unit. Then, it is possible to display the sensed pressure data on an external evaluation unit without making it necessary to connect the implantation catheter with an electric line to this external evaluation unit. By integrating the wireless communication module into the handle, it is not necessary to increase the size of the catheter shaft of the implantation catheter. Rather, the catheter shaft can be dimensioned as small as a catheter shaft of implantation catheters known from prior art. The (electronic) pressure sensor usually does not necessitate much space. All parts and modules that need more space can be fully integrated into the handle of the implantation catheter. Furthermore, a wireless connection between the handle of such an implantation catheter to an external evaluation unit is typically more easily established and more reliably maintained than a wireless communication connection between an external evaluation unit and a catheter shaft or a tip of a catheter shaft currently present within the body of a human or animal patient. In an embodiment, the pressure sensor of the implantation catheter is not realized by an electronic pressure sensor, but rather in the form of a lumen having a distal opening at the distal end of the catheter shaft. Thereby, the measuring lumen is designed and arranged to be filled with a liquid. The liquid column being present in the measuring lumen then directly transfers the vascular pressure being present at the distal opening of the measuring lumen to the pressure measuring device connected to a proximal opening of the measuring lumen. This pressure measuring device can be integrated into a handle of the catheter shaft. Furthermore, it is possible to close the distal opening of the measuring lumen with a membrane through which pressure can be transferred onto a liquid column being present in the measuring lumen, wherein the membrane prevents the liquid being present in the measuring lumen from exiting the measuring lumen through the distal opening. If the pressure at the distal end of the catheter shaft is measured with the help of such a liquid-filled measuring lumen, no electronic components need to be present at the tip of the catheter shaft.

In an embodiment, the vascular implant is a sensory implant. In a further embodiment, the vascular implant is or comprises a pressure sensor, a temperature sensor, an oxygen saturation sensor, a pH sensor, a PCO2 sensor, a PO2 sensor, a glucose sensor, and/or a flow sensor. If the vascular implant is implanted into the pulmonary artery, it is then possible to measure the pulmonary pressure, the temperature of the blood flowing through the pulmonary artery and/or the flow of blood flowing through the pulmonary artery and/or other parameters related or unrelated to the cardiopulmonary system. E.g. pulmonary artery oxygen saturation may be to estimate cardiac output.

In an embodiment, the vascular implant comprises a data transfer unit for transferring sensed data to an external evaluation unit. With the help of such a data transfer unit, it is possible to read out the vascular implant in a wireless manner. Then, it is particularly easy to get access to the data sensed by the vascular implant.

In an embodiment, the vascular implant is arranged on an outside of the catheter shaft. To give an example, the vascular implant can abut on the outside of the catheter shaft such that the diameter of the catheter shaft is only minimally increased by the vascular implant.

In another embodiment, the vascular implant is arranged inside the catheter shaft. Then, the vascular implant is particularly well protected against external influences during advancement of the implantation catheter through the vascular system of the patient. Thereby, it is possible to cover the vascular implant with a protective covering. It is also possible that the catheter shaft or a part of the catheter shaft (such as an outer catheter shaft) serves as protective covering for the vascular implant. If the covering or the outer catheter shaft, respectively, are retracted with respect to the vascular implant, the vascular implant is released from the catheter shaft at the desired implantation site.

In another aspect, an implantation catheter arrangement comprising an implantation catheter according to the preceding explanations and an evaluation unit is provided. Thereby, the evaluation unit is operatively coupled with the data transfer unit of the vascular implant and with the pressure sensor of the catheter. Thus, the evaluation unit is designed and arranged to receive data from the pressure sensor of the implantation catheter and from the vascular implant. Furthermore, the evaluation unit has a display for displaying the data received from the pressure sensor and from the vascular implant at the same time. Thus, the evaluation unit makes it possible to display a signal of the vascular implant and a signal of the pressure sensor arranged on the implantation catheter collectively so that a comparison of both signals is particularly easy. Furthermore, if zero adjustments are necessary, such adjustments can also be particularly easy performed if both signals are displayed at the same time. This significantly facilitates the implantation of a vascular implant, such as a pressure sensor, into the vessel (e.g. the pulmonary artery). Therefore, even medical doctors having only little experience in implanting vascular implants into the pulmonary artery or into another vessel are thus enabled to implant the vascular implant with the help of the implantation catheter according to the present disclosure in a particularly simple and safe way.

Generally, the evaluation unit can be operatively coupled with the pressure sensor of the catheter by means of a data transfer line. In an embodiment, however, the operative coupling is established by a wireless connection between the evaluation unit and the pressure sensor of the catheter or a wireless communication module of an evaluation electronics being, e.g., present in the handle of the implantation catheter. Furthermore, the operative coupling between the evaluation unit and a data transfer unit of the vascular implant is typically established by means of a wireless data connection. If both the coupling between the evaluation unit and the vascular implant and the coupling between the evaluation unit and the pressure sensor is established by means of wireless connections, the handling of the implantation catheter arrangement is particularly simple. Then, it is possible that a medical doctor handles the implantation catheter without being restricted by any electric lines connecting the implantation catheter with the evaluation unit. Rather, the evaluation unit can be freely positioned at a desired location independent on the exact positioning of the implantation catheter.

The implantation catheter of the present invention can be implanted into a vessel (e.g. the pulmonary artery) of a human or an animal patient by an implantation method not according to the present invention comprising the steps explained in the following.

First, the balloon is inflated. This is typically done with a liquid such as an isotonic salt solution. The volume of liquid fed into the balloon determines the expansion of the balloon and thus the final size of the balloon.

Then, the catheter is advanced towards an intended implantation site within the vessel (e.g. of the patient's heart). Thereby, the catheter can be advanced until the balloon gets stuck in the pulmonary artery. Thus, the size of the balloon determines how far the catheter can be advanced into the pulmonary artery.

The balloon is then kept in its inflated state and the pulmonary wedge pressure is measured to obtain a first value of the pulmonary wedge pressure.

Additionally or alternatively, the balloon is deflated and the pulmonary artery pressure is measured to obtain a first value of the pulmonary artery pressure.

Afterwards, the vascular implant is released from the implantation catheter. It is then implanted at the intended implantation site.

Afterwards, the balloon is inflated again (if it was deflated beforehand) or is kept in its inflated state (if it was in that inflated state before). Then, the pulmonary wedge pressure is measured for a second time to obtain a second value of the pulmonary wedge pressure.

Additionally or alternatively, the balloon is kept in its deflated state (if it was deflated beforehand) or the balloon is deflated (if it was in its inflated state beforehand). Then, the pulmonary artery pressure is measured for a second time to obtain a second value of the pulmonary artery pressure.

The first value of the pulmonary wedge pressure and the second value of the pulmonary wedge pressure can then be compared. Likewise, the first value of the pulmonary artery pressure and the second value of the pulmonary artery pressure can be compared. Typically these comparisons of the first and second values of the respective pressure should result in no significant deviations between the first and second values. If a significant deviation between the first and second values is determined, this indicates that the implanted vascular implant may be responsible for the measured different pressure. This can be an indication that the vascular implant is wrongly placed and obstructs a vessel. Then, the implant needs to be removed and re-implanted to avoid such undesired vessel obstruction.

In an embodiment, the balloon is deflated prior to releasing the vascular implant from the implantation catheter. By such a deflation of the balloon, the release of the vascular implant from the implantation catheter is facilitated. Depending on the nature of the vascular implant and the concrete arrangement of the balloon arranged around the outside of the catheter shaft, such deflation might not be necessary in each case.

The method can be carried out without using another catheter for measuring the pulmonary wedge pressure or the pulmonary artery pressure. Thus, the method can take advantage of the properties of the presently disclosed implantation catheter, namely the possibility of refraining from using a second catheter for pressure measurements and to use only one single device for implanting the vascular implant and for measuring the relevant hemodynamic parameters such as the pulmonary wedge pressure.

The features disclosed herein in regard to the implantation catheter can also be applied to the implantation arrangement and vice versa.

Further details will be explained in the following with respect to exemplary embodiments and accompanying Figures. In the Figures:
- Fig. 1: shows a longitudinal section through an implantation catheter known from prior art;
- Fig. 2: shows a schematic sketch of a first embodiment of an implantation catheter comprising an inflatable balloon;
- Fig. 3: shows a schematic sketch of a second embodiment of an implantation catheter comprising an inflatable balloon; and
- Fig. 4: shows a schematic sketch of a third embodiment of an implantation catheter comprising an inflatable balloon.

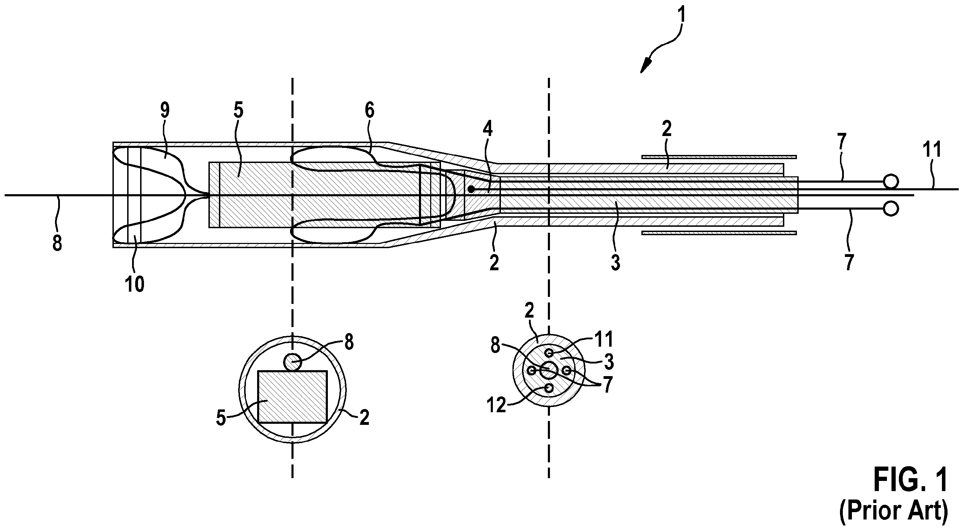

Figure 1 shows a longitudinal section of an implantation catheter 1 according to prior art. This implantation catheter 1 comprises an outer catheter and an inner catheter 3. The inner catheter 3 comprises a pushing element 4 at its distal end. This pushing element 4 serves for pushing an implant 5 out of the implantation catheter 1 once the desired implantation site has been reached. For securing the implant 5 within the implantation catheter 1, a proximal fixation loop 6 is provided that can be moved by pulling two fixation wires 7.

The implantation catheter is guided on a guide wire 8 through the body of the patient towards the intended implantation site. In order to locate the desired implantation site and the correct positioning of the implantation catheter 1, the implant 5 is provided with an antenna 9 and an X-ray opaque marker 10 arranged at the antenna 9.

For steering the implantation catheter 1 through the body of the human or animal patient, a pull wire 11 is provided that is tightly connected with the pushing element 4 of the inner catheter 3.

At the desired implantation site, the outer catheter 2 is retracted with respect to the inner catheter 3 so as to release the implant 5 from the implantation catheter 1.

Below the longitudinal section of Figure 1, two cross-sectional views (along the dashed lines through the longitudinal section) are provided. The left cross-sectional view shows the relative arrangement of the guidewire 8 and the implant 5 inside the outer catheter 2 in a distal end section of the implantation catheter 1. The right cross-sectional view shows the different lumina that are present in the inner catheter 3 through which the fixation wires 7, the pull wire 11 for steering the implantation catheter 1 and the guidewire 8 are guided. Furthermore, an empty lumen 12 can be seen that is not needed for the functionality of the implantation catheter 1.

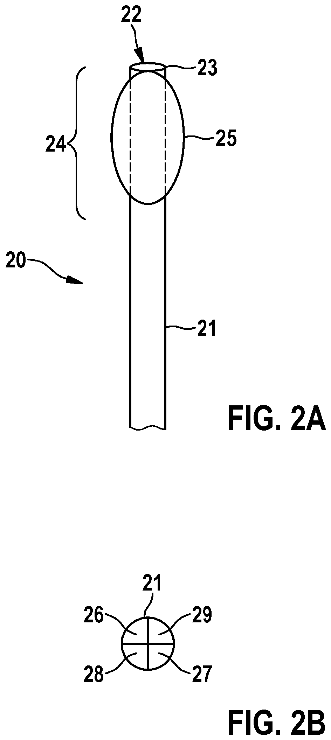

Figure 2A shows an implantation catheter 20 comprising a catheter shaft 21 with a distal opening 22 at its distal end 23. The catheter shaft 21 is arranged and designed to house an implant in its interior or on its outside.

In a distal end section 24 of the catheter shaft 21, a balloon 25 is provided that radially extends around an outside of the catheter shaft 21. Due to this balloon 25, the implantation catheter 20 can float with the bloodstream towards a desired site of implantation, e.g., inside the pulmonary artery.

Figure 2B shows a cross-sectional view through the implantation catheter 20 of Figure 2A. Thereby, the same numeral references will be used in this and in the following Figures for the same structural elements.

The catheter shaft 21 comprises a first lumen 26 and a second lumen 27 for guiding fixation wires for fixing an implant arranged inside the catheter shaft 21. It furthermore comprises a balloon lumen 28 that serves for providing the balloon 25 with an appropriate liquid such as a salt solution for inflating the balloon 25. The catheter shaft 21 furthermore comprises a measuring lumen 29 that has a distal opening and is filled with a liquid. The pressure being present on the distal end 23 of the implantation catheter 20 can then be transferred through the liquid column being present in the measuring lumen 29 to a proximal end of the implantation catheter 20 and a pressure monitoring device being arranged at the proximal end of the implantation catheter 20. Thus, the measuring lumen 29 serves - together with the pressure monitoring device additionally provided at the proximal end of the measuring lumen 29 - as pressure sensor of the implantation catheter 20.

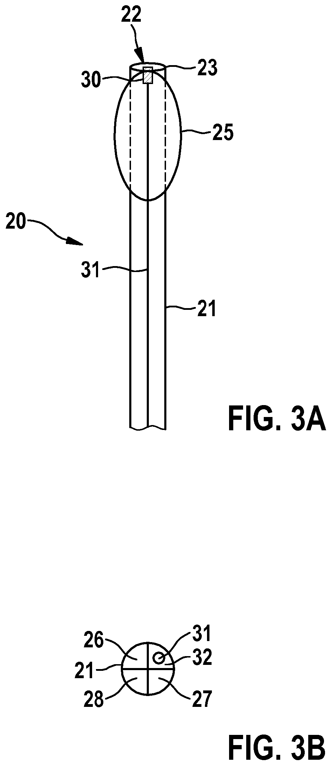

Figure 3A shows another embodiment of the implantation catheter 20, wherein reference is made to the explanations given with respect to Figures 2A and 2B. In the following, only the differences to the embodiment of Figures 2A and 2B will be explained.

In contrast to the implantation catheter 20 depicted in Figure 2A, the implantation catheter 20 of Figure 3A has an electronic pressure sensor 30 arranged at the distal end 23 of the implantation catheter 20. This electronic pressure sensor 30 is connected via a connecting line 31 with an evaluation unit being present at a proximal end of the catheter shaft 21, e.g., in a handle of the implantation catheter 20. Then, it is possible to transmit signals sensed by the electronic pressure sensor 30 through the connecting line 31 towards such an evaluation electronics.

Figure 3B shows a cross-sectional view through the implantation catheter 20 of Figure 3A. Once again, a first lumen 26 and a second lumen 27 for guiding fixation wires for fixing an implant being present within the catheter shaft 21 are provided. Furthermore, a balloon lumen 28 is provided through which a salt solution can be fed to the balloon 25 in order to inflate the balloon 25. Likewise, the balloon lumen 28 serves for withdrawing such a salt solution from the balloon 25 in order to deflate the balloon.

The implantation catheter 20 of Figure 3B uses a measuring lumen 32 for guiding the connecting line 31 that connects the electronic pressure sensor 30 with an evaluation electronics. Thus, in contrast to the exemplary embodiment depicted in Figures 2A and 2B, the measuring lumen 32 is not filled with a liquid like the measuring lumen 29, but rather serves for guiding the connecting line 31.

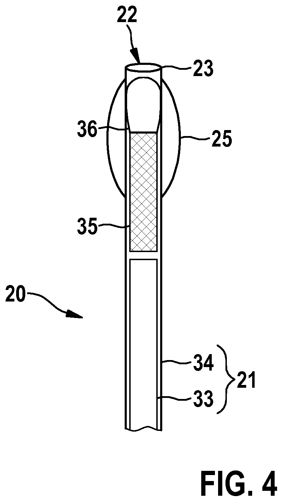

Figure 4 shows a longitudinal section through another embodiment of the implantation catheter 20. It comprises an inner catheter 33 and an outer catheter 34. Both serve as catheter shaft 21. An implant 35 is located distal of the inner catheter 33. The outer catheter 34 serves as protective covering for the implant 35. The balloon 25 is arranged outside the outer catheter 34 circumferentially around the outer catheter 34. Distal of the implant 35, a fixation structure 36 is present inside the outer catheter 34 that serves for fixing the implant 35 inside the catheter shaft 21. Once the implant 35 is to be released from the implantation catheter 20, the fixation structure 36 is retracted. At the same time, the outer catheter 34 is retracted relatively with respect to the inner catheter 33. Then, the implant 35 is released and can be implanted at the desired site of implantation.

## Claims

1. An implantation catheter for implanting a vascular implant into a vessel of a human or animal, comprising a catheter shaft (21) having a proximal end and a distal end (23), a vascular implant (35) carried by the catheter shaft (21), and a pressure sensor (29; 30) for sensing a vascular pressure at the distal end (23), wherein the implantation catheter (20) comprises an inflatable balloon (25) arranged circumferentially around an outside of the catheter shaft (21) in a distal end section (24) of the catheter shaft, **characterized in that** a distal end of the balloon (25) flushes with the distal end (23) of the catheter shaft (21).

2. The implantation catheter according to claim 1, wherein the distal end section (24) extends from the distal end (23) towards the proximal end over up to 10% of the total length of the catheter shaft (21).

3. The implantation catheter according to any of the preceding claims, wherein the pressure sensor is an electronic pressure sensor (30).

4. The implantation catheter according to claim 3, wherein the implantation catheter comprises a handle, wherein the handle comprises an evaluation electronics for reading the pressure sensor (30) out.

5. The implantation catheter according to claim 3 or 4, wherein the evaluation electronics comprises a wireless communication module for transferring pressure data sensed by the pressure sensor (30) to an external evaluation unit.

6. The implantation catheter according to any of claims 1 or 2, wherein the pressure sensor comprises a measuring lumen (29) having a distal opening at the distal end (23) of the catheter shaft (21), wherein the measuring lumen (29) is designed and arranged to be filled with a liquid through which a vascular pressure outside the distal opening of the measuring lumen (29) can be transferred to a pressure measuring device connected to a proximal opening of the measuring lumen (29).

7. The implantation catheter according to any of the preceding claims, wherein the vascular implant (35) is or comprises at least one of a pressure sensor, a temperature sensor and a flow sensor.

8. The implantation catheter according to any of the preceding claims, wherein the vascular implant (35) comprises a data transfer unit for transferring sensed data to an external evaluation unit.

9. The implantation catheter according to any of the preceding claims, wherein the vascular implant (35) is arranged on an outside of the catheter shaft (21).

10. The implantation catheter according to any of claims 1 to 8, wherein the vascular implant (35) is arranged inside the catheter shaft (21).

11. An implantation catheter arrangement comprising an implantation catheter (20) according to any of the preceding claims and an evaluation unit, wherein the evaluation unit is operatively coupled with a data transfer unit of the vascular implant (35) and with the pressure sensor (29; 30) of the implantation catheter (20), wherein the evaluation unit is designed and arranged to receive data from the pressure sensor (29; 30) and from the vascular implant (35), wherein the evaluation unit has a display for displaying the data received from the pressure sensor (29; 30) and from the vascular implant (35) at the same time.

## Patentansprüche

1. Implantationskatheter zum Implantieren eines Gefäßimplantats in ein Gefäß eines Menschen oder Tieres, einen Katheterschaft (21) mit einem proximalen Ende und einem distalen Ende (23), ein Gefäßimplantat (35), das vom Katheterschaft (21) transportiert wird, und einen Drucksensor (29; 30) zum Erfassen eines Gefäßdrucks am distalen Ende (23) umfassend, wobei der Implantationskatheter (20) einen aufblasbaren Ballon (25) umfasst, der umlaufend um eine Außenseite des Katheterschafts (21) in einem distalen Endabschnitt (24) des Katheterschafts angeordnet ist, **dadurch gekennzeichnet, dass** ein distales Ende des Ballons (25) bündig mit dem distalen Ende (23) des Katheterschafts (21) ist.

2. Implantationskatheter nach Anspruch 1, wobei der distale Endabschnitt (24) vom distalen Ende (23) in Richtung des proximalen Endes über bis zu 10 % der Gesamtlänge des Katheterschafts (21) verläuft.

3. Implantationskatheter nach einem der vorstehenden Ansprüche, wobei der Drucksensor ein elektronischer Drucksensor (30) ist.

4. Implantationskatheter nach Anspruch 3, wobei der Implantationskatheter einen Griff umfasst, wobei der Griff eine Auswerteelektronik zum Auslesen des Drucksensors (30) umfasst.

5. Implantationskatheter nach Anspruch 3 oder 4, wobei die Auswerteelektronik ein drahtloses Kommunikationsmodul zur Übertragung von Druckdaten, die der Drucksensor (30) erfasst, an eine externe Auswerteeinheit umfasst.

6. Implantationskatheter nach einem der Ansprüche 1 oder 2, wobei der Drucksensor ein Messlumen (29) mit einer distalen Öffnung am distalen Ende (23) des Katheterschafts (21) umfasst, wobei das Messlumen (29) dafür ausgelegt und angeordnet ist, mit einer Flüssigkeit befüllt zu werden, durch die ein Gefäßdruck außerhalb der distalen Öffnung des Messlumens (29) an eine Druckmessvorrichtung übertragen werden kann, die mit einer proximalen Öffnung des Messlumens (29) verbunden ist.

7. Implantationskatheter nach einem der vorstehenden Ansprüche, wobei das Gefäßimplantat (35) mindestens eins von einem Drucksensor, einem Temperatursensor und einem Durchflusssensor ist oder umfasst.

8. Implantationskatheter nach einem der vorstehenden Ansprüche, wobei das Gefäßimplantat (35) eine Datenübertragungseinheit zur Übertragung erfasster Daten an eine externe Auswerteeinheit umfasst.

9. Implantationskatheter nach einem der vorstehenden Ansprüche, wobei das Gefäßimplantat (35) an einer Außenseite des Katheterschafts (21) angeordnet ist.

10. Implantationskatheter nach einem der Ansprüche 1 bis 8, wobei das Gefäßimplantat (35) im Katheterschaft (21) angeordnet ist.

11. Implantationskatheter-Anordnung, einen Implantationskatheter (20) nach einem der vorstehenden Ansprüche und eine Auswerteeinheit umfassend, wobei die Auswerteeinheit in Wirkbeziehung mit einer Datenübertragungseinheit des Gefäßimplantats (35) und mit dem Drucksensor (29; 30) des Implantationskatheters (20) gekoppelt ist, wobei die Auswerteeinheit dafür ausgelegt und angeordnet ist, Daten vom Drucksensor (29; 30) und vom Gefäßimplantat (35) zu empfangen, wobei die Auswerteeinheit ein Display zur gleichzeitigen Anzeige der vom Drucksensor (29; 30) und vom Gefäßimplantat (35) empfangenen Daten hat.

## Revendications

1. Cathéter d'implantation pour implanter un implant vasculaire dans un vaisseau d'un humain ou d'un animal, comprenant une tige de cathéter (21) ayant une extrémité proximale et une extrémité distale (23), un implant vasculaire (35) porté par la tige de cathéter (21), et un capteur de pression (29, 30) pour détecter une pression vasculaire à l'extrémité distale (23), où le cathéter d'implantation (20) comprend un ballonnet (25) gonflable disposé de manière circonférentielle autour d'un extérieur de la tige de cathéter (21) dans un segment d'extrémité distale (24) de la tige de cathéter, **caractérisé en ce qu'**une extrémité distale du ballonnet (25) affleure avec l'extrémité distale (23) de la tige de cathéter (21).

2. Cathéter d'implantation selon la revendication 1, dans lequel le segment d'extrémité distale (24) s'étend de l'extrémité distale (23) vers l'extrémité proximale jusqu'à plus de 10 % de la longueur totale de la tige de cathéter (21).

3. Cathéter d'implantation selon l'une quelconque des revendications précédentes, dans lequel le capteur de pression est un capteur de pression électronique (30).

4. Cathéter d'implantation selon la revendication 3, dans lequel le cathéter d'implantation comprend une poignée, où la poignée comprend une électronique d'évaluation pour lire le contenu du capteur de pression (30).

5. Cathéter d'implantation selon la revendication 3 ou la revendication 4, dans lequel l'électronique d'évaluation comprend un module de communication sans fil pour transférer des données de pression détectées par le capteur de pression (30) vers une unité d'évaluation extérieure.

6. Cathéter d'implantation selon l'une quelconque des revendications 1 ou 2, dans lequel le capteur de pression comprend une lumière de mesure (29) ayant une ouverture distale à l'extrémité distale (23) de la tige de cathéter (21), où la lumière de mesure (29) est prévue et arrangée pour être remplie avec un liquide à travers lequel une pression vasculaire à l'extérieur de l'ouverture distale de la lumière de mesure (29) peut être transférée vers un dispositif de mesure de la pression connecté à une ouverture proximale de la lumière de mesure (29).

7. Cathéter d'implantation selon l'une quelconque des revendications précédentes, dans lequel l'implant vasculaire (35) est ou comprend au moins un capteur parmi un capteur de pression, un capteur de température ou un capteur de flux.

8. Cathéter d'implantation selon l'une quelconque des revendications précédentes, dans lequel l'implant vasculaire (35) comprend une unité de transfert de données pour transférer des données détectées vers une unité d'évaluation extérieure.

9. Cathéter d'implantation selon l'une quelconque des revendications précédentes, dans lequel l'implant vasculaire (35) est disposé sur l'extérieur de la tige de cathéter (21).

10. Cathéter d'implantation selon l'une quelconque des revendications 1 à 8, dans lequel l'implant vasculaire (35) est disposé à l'intérieur de la tige de cathéter (21).

11. Agencement de cathéter d'implantation comprenant un cathéter d'implantation (20) selon l'une quelconque des revendications précédentes et une unité d'évaluation, où l'unité d'évaluation est couplée opérationnellement avec une unité de transfert de données de l'implant vasculaire (35) et avec le capteur de pression (29 ; 30) du cathéter d'implantation (20), où l'unité d'évaluation est prévue et arrangée pour recevoir des données du capteur de pression (29 ; 30) et de l'implant vasculaire (35), où l'unité d'évaluation possède un affichage pour afficher en même temps les données reçues du capteur de pression (29 ; 30) et de l'implant vasculaire (35).
